(19) Europäisches Patentamt
European Patent Office
Office européen des brevets

(11) Veröffentlichungsnummer: **0 388 510 A1**

(12) # EUROPÄISCHE PATENTANMELDUNG

(21) Anmeldenummer: **89115520.2**

(22) Anmeldetag: **23.08.89**

(51) Int. Cl.$^5$: **A61K 37/02**

(30) Priorität: **18.03.89 DE 3909056**

(43) Veröffentlichungstag der Anmeldung:
**26.09.90 Patentblatt 90/39**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Seibert, Gerhard, Prof.-Dr.**
**Gläserweg 21**
**D-6100 Darmstadt(DE)**
Erfinder: **Isert, Dieter, Dr.**
**Hamburger Strasse 1**
**D-6236 Eschborn(DE)**
Erfinder: **Klesel, Norbert, Dr.**
**Bornstrasse 50**
**D-6238 Hofheim am Taunus(DE)**

(54) **Pharmazeutische Kombinationspräparate und deren Verwendung zur Prophylaxe oder Behandlung bakterieller Infektionskrankheiten.**

(57) Decaplanin und Cephalosporinderivate zeigen gemeinsam verabreicht eine synergistische antibakterielle Wirkung. Entsprechende Kombinationspräparate eignen sich deshalb zur Prophylaxe und Behandlung bakterieller Infektionskrankheiten.

EP 0 388 510 A1

# Pharmazeutische Kombinationspräparate und deren Verwendung zur Prophylaxe oder Behandlung bakterieller Infektionskrankheiten

Die vorliegende Erfindung betrifft pharmazeutische Kombinationspräparate und deren Verwendung zur Prophylaxe oder Behandlung bakterieller Infektionskrankheiten.

Es ist bekannt, daß sich Antibiotika aus der Klasse der Cephalosporine, wie z.B. Cefpirom, hervorragend zur Therapie bakterieller Infektionen eignen, daß jedoch die antibakterielle Aktivität gegenüber bestimmten grampositiven und gramnegativen Keimen, insbesondere Staphylococcus-Arten, nicht ausreichend sein kann. Infektionen mit Staphylokokken, die Methicillin-Resistenz zeigen (MRSA, MRSE), fallen üblicherweise nicht in die Indikation von Cephalosporinen. Antibiotika aus der Gruppe der Glykopeptide, z.B. Vancomycin, haben eine antibakterielle Aktivität, die viele Staphylokokken-Stämme abtötet, gegen die Cephalosporine nur wenig wirksam sind.

Es wurde nun gefunden, daß Cephalosporinderivate in Kombination mit dem Glykopeptid-Antibiotikum Decaplanin einen überraschenderweise deutlichen synergistischen Effekt gegenüber Methicillin-resistenten Staphylokokken haben. Synergistische Wirkungen von ß-Lactam-Antibiotika mit anderen Glykopeptiden, wie z.B. Vancomycin, sind beschrieben in H. Portier et al., Infection 13/1 (1985) 123-128. Überraschenderweise ist jedoch der synergistische Effekt von Cephalosporinderivaten mit Decaplanin gegenüber Methicillin-resistenten Staphylokokken sehr viel stärker als dies mit Vancomycin gefunden werden kann. Er kann außerdem mit allen Stämmen gefunden werden, während z.B. Vancomycin und Cefpirom nur mit manchen Staphylokokken-Stämmen diesen Effekt zeigen.

Die vorliegende Erfindung betrifft demzufolge ein pharmazeutisches Kombinationspräparat enthaltend Decaplanin, eine Verbindung der Formel A,

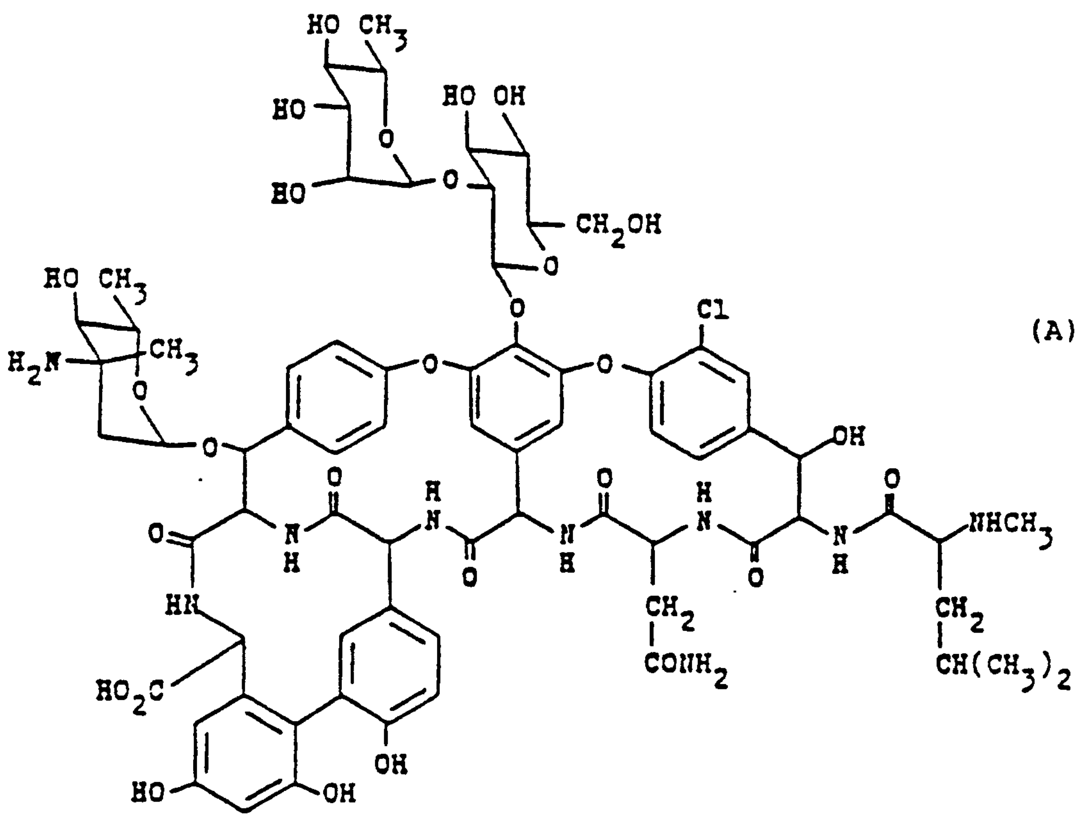

(A)

oder deren physiologisch verträgliche Salze und ein Cephalosporinderivat oder dessen physiologisch verträgliche Salze oder Ester.

Decaplanin wird hergestellt durch Kultvierung des Mikroorganismus Y 8636 910 (hinterlegt bei der Deutschen Sammlung von Mikroorganismen am 5. August 1988, unter der Nummer DSM 4763) wobei das Decaplanin nach bekannten Methoden isoliert wird (vgl. europäische Patentanmeldung 88 114 022.2).

Die Eigenschaften von Decaplanin sind in der europäischen Patentanmeldung 88 114 022.2 beschrieben.

Die Herstellung und die Eigenschaften von Cephalosporinderivaten sind z.B. beschrieben in den Deutschen Offenlegungsschriften 27 02 501, 27 13 272, 27 15 385, 28 10 922, 29 21 316, 29 22 036, in der EP 00 64 740, in der U.S. 4 278 793 oder der U.S. 4 501 739, in der GB 2 105 334 oder der GB 2 105 335.
Die bevorzugten physiologisch verträglichen Salze und Ester sind ebenfalls in den genannten Druckschriften aufgeführt.

Bevorzugte Cephalosporinderivate sind diejenigen der Formel I

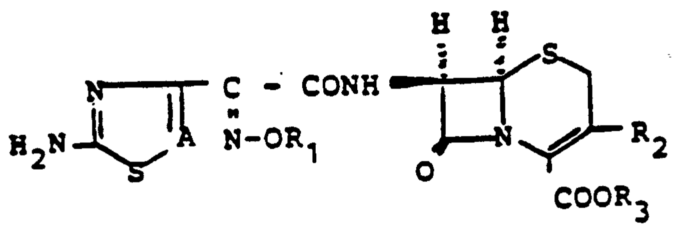

(I)

in der A CH oder N bedeutet und
$R_1$ die Bedeutung besitzen kann von Wasserstoff, $C_1$-$C_4$-Alkyl, Carboxy-$C_1$-$C_4$-alkyl oder einer Gruppe der Formel

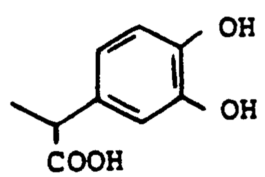

und in der die Gruppe $=N\text{-}OR_1$ in syn-Position steht,
$R_2$ die Bedeutung haben kann von Wasserstoff, Methyl, Methoxy, Vinyl, Acetoxymethyl, Carbamoyloxymethyl, von

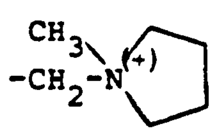,

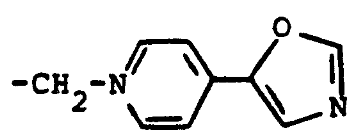,

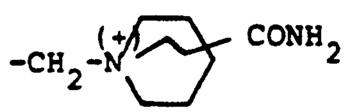

oder von

$-CH_2S-X$,

mit X = 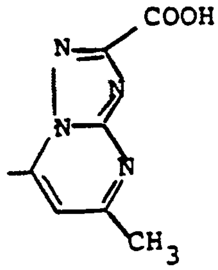 ,

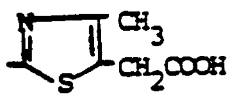 , 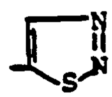 , 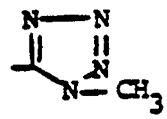 , 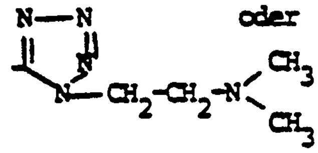 oder 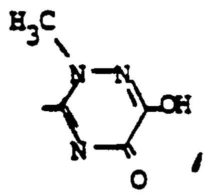 ,

von 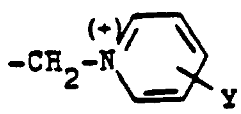

mit Y = Wasserstoff,
$C_1$-$C_4$-Alkylthio,
$C_1$-$C_4$-Alkoxy oder
$C_3$-$C_5$-Cycloalkyl,

von 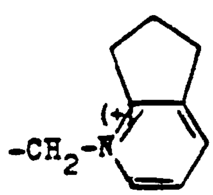 oder 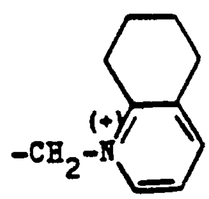

wobei die ankondensierten Ringe auch in 3,4-Stellung stehen und auch durch Sauerstoff unterbrochen sein können, von

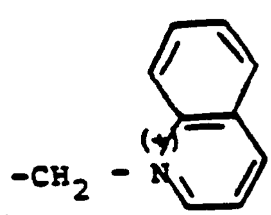 oder 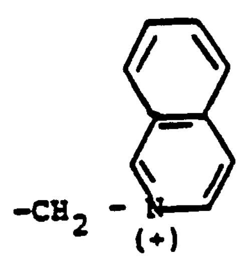

von Thienopyridinio-methyl, Furopyridinio-methyl oder von 5-Methyl-tetrazol-2-yl-methyl und
$R_3$ für Wasserstoff, ein physiologisch verträgliches Kation, einen physiologisch vertraglichen Esterrest oder - falls in $R_2$ die Struktur

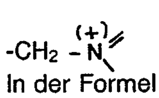 auftritt - für eine negative Ladung stehen kann.

In der Formel 1 bedeutet A vorzugsweise CH.

Steht $R_1$ für $C_1$-$C_4$-Alkyl, so kommen z.B. Methyl, Ethyl oder Propyl in Betracht, vorzugsweise Methyl.

Steht $R_1$ für Carboxy-$C_1$-$C_4$-alkyl, so sind z.B. Carboxymethyl, Carboxyethyl oder Carboxypropyl, vorzugsweise der Rest $-CH_2-COOH$, insbesondere jedoch der Rest

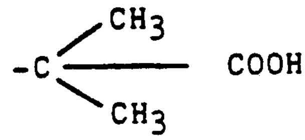

von Interesse.

Von den vorstehend aufgeführten substituierten Pyridiniummethyl-Resten sind bevorzugt 2,3-Cyclopenteno- und 2,5-Cyclohexeno-pyridinium-methyl, sowie 4-Methylthio-, 4-Cyclopropyl- und 3-Methoxy-pyridinium-methyl, sowie ebenfalls 3,4-Cyclopenteno- und 3,4-Cyclohexeno-pyridinium-methyl.

Erfindungsgemäß von ganz besonderem Interesse sind diejenigen Verbindungen der Formel I, in der $R_1$ für Methyl und $R_2$ für $-CH_2-OCOCH_3$ (Cefotaxim),

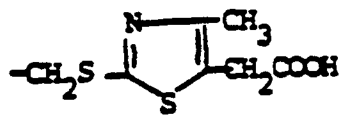
(Cefodizim),

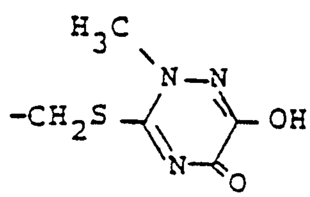
(Ceftriaxon),

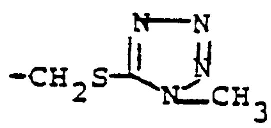
(Cefmenoxim) oder

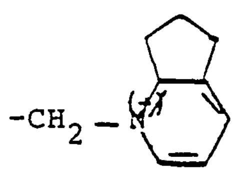
(Cefpirom) steht, oder

$R_1$ für $-CH_2COOH$ und $R_2$ für $-CH=CH_2$ (Cefixim) steht, wobei innerhalb dieser Gruppe das Cefodizim, Cefpirom, Cefotaxim, Ceftriaxon nochmals eine Vorzugsstellung einnehmen.

Steht $R_2$ für eine $-CH_2$-Pyridinium-Verbindung, so liegt in der allgemeinen Formel I die Carboxylgruppe als Anion eines inneren Salzes ($-COO^{(-)}$) vor.

$R_3$ kann stehen für Wasserstoff oder ein physiologisch verträgliches Kation, wie z.B. ein Alkalikation, vorzugsweise Kalium oder Natrium, insbesondere Natrium, oder auch andere literaturbekannte, physiologisch verträgliche Kationen wie z.B. Erdalkali- oder organische Ammoniumionen (vgl. z.B. U.S. 4 278 793).

$R_3$ kann weiterhin stehen für einen insbesondere für die enterale Applikation interessanten, physiologisch verträglichen Esterrest, wie z.B. für einen Acyloxymethyl-oder Acyloxyethylrest mit 2 bis 12, vorzugsweise 2 bis 6 C-Atomen im Acylteil, vorzugsweise Acetoxymethyl, 1′-(Acetoxy)ethyl oder Pivaloyloxymethyl, für 5-Methyl-1,3-dioxalen-2-on-4-yl-methyl, oder auch für andere, physiologisch verträgliche Esterreste, wie sie beispielsweise in der EP-A 0170028 beschrieben sind.

Als besonders interessante Cephalosporinderivate seien ferner erwähnt unter den Aminothiazolcephalosporinen, die $-CH_2-$ anstelle von $-C(=N-OR_1)$-enthalten, das Cefotiam und unter den N-Acyl-phenylglycin-cephalosporinen das Cefoperazon (vgl. z.B. EP-0 248 361).

Weitere Beispiele für erfindungsgemäß bevorzugte Cephalosporinderivate sind ausgewählt aus der Gruppe der folgenden Verbindungen:

Cefpirom, eine Verbindung der Formel

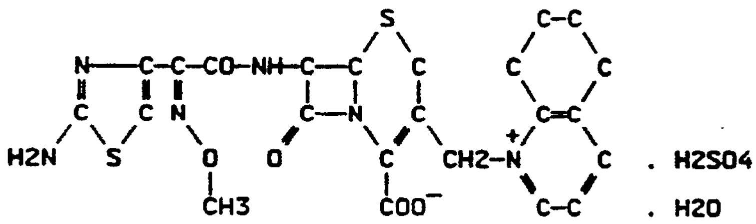

Cefuroxim, eine Verbindung der Formel

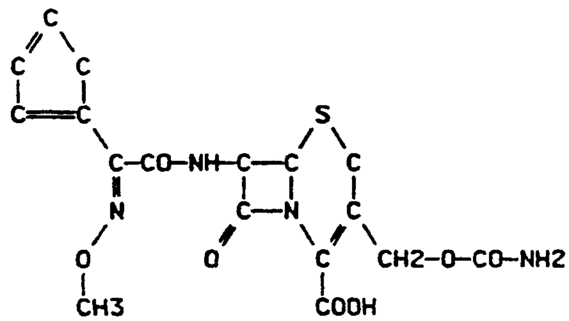

Ceftizoxim, eine Verbindung der Formel

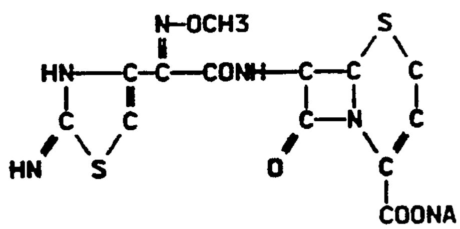

und Ceftazidim, eine Verbindung der Formel

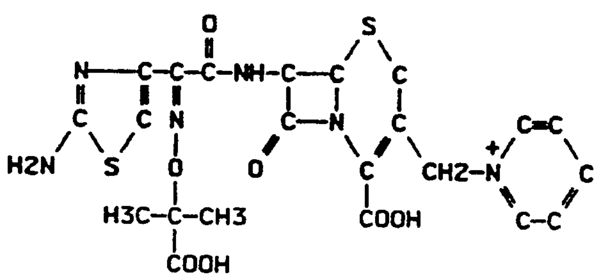

Weitere besonders bevorzugte Cephalosporinderivate sind ausgewählt aus der Gruppe der folgenden Verbindungen:

Cefepim, eine Verbindung der Formel

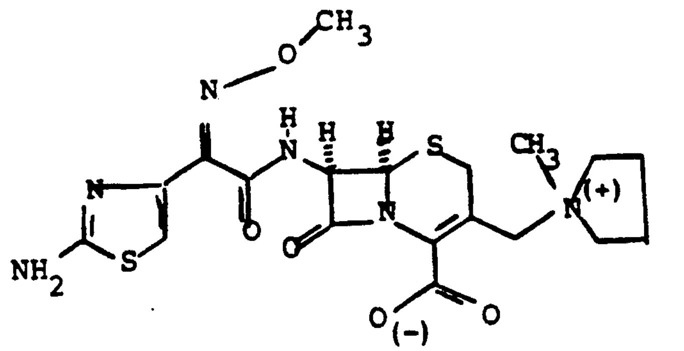

,

7- [(2-Amino- thiazol-4-yl)-methoxyimino- acetamido]- 3- [4-(oxazol-5-yl)- 1-pyrimidiniomethyl]-8-oxo-5-thia-1-azabicyclo[4.2.0]oct-2-en-2-carboxylat, eine Verbindung der Formel

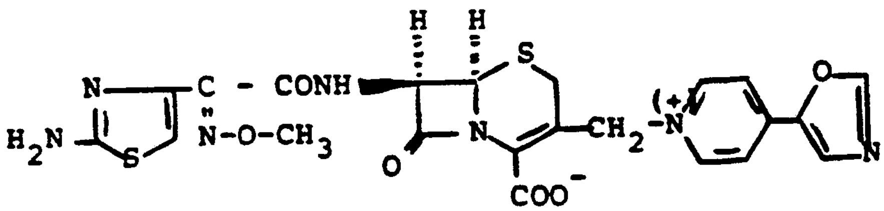

(6R,7R)-7-[(2-Amino-thiazol-4-yl)-((Z)-(S)-α-carboxy-3,4-dihydroxy-benzyloxyimino)-acetamido]-3-[(2-carboxy-5-methyl-S-triazolo[1,5-a]-pyrimidin-7-yl)-thiomethyl]-8-oxo-5-thia-1-azabicyclo[4.2.0]oct-2-en-2-carbonsäure, eine Verbindung der Formel

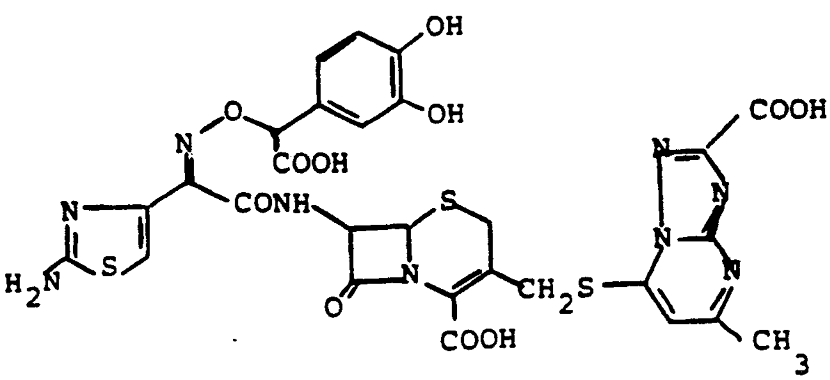

und

(6R,7R)-7-[(5-Amino-1,2,4-thiadiazol-3-yl)- (Z)-methoxyiminoacetamido]-3-[4-carbamoyl-1-chinuclidiniomethyl]-8-oxo-5-thia-1-azabicyclo[4.2.0]oct-2-en-2-carboxylat, eine Verbindung der Formel

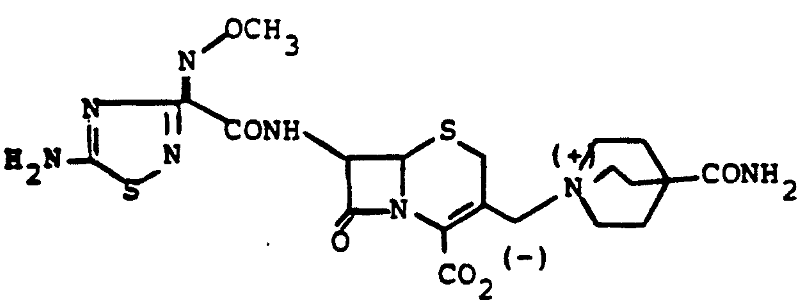

sowie deren physiologisch verträgliche Salze oder Ester.

Ein ganz besonders bevorzugtes Cephalosporinderivat ist Cefpirom, eine Verbindung der Formel

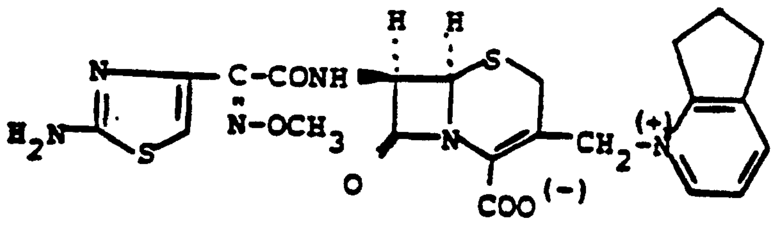

Die Herstellung der erfindungsgemäß in Betracht kommenden Komponenten der Wirkstoffkombination wird beispielsweise in den vorstehend genannten Druckschriften beschrieben.

Die erfindungsgemäße Kombination von Cephalosporinen und Decaplanin wirkt stark antibakteriell und ist deshalb zur Behandlung bakterieller Infektionen besonders gut geeignet. Wesentlich ist die Tatsache, daß die Wirkung der beiden Komponenten sich nicht additiv verhält, sondern vielmehr ein unerwarteter, stark synergistischer Effekt auftritt.

Selbst bei Keimen, gegen die eine Einzelkomponente allein keine signifikante antibakterielle Wirkung hat, ist in Kombination ein synergistischer Effekt zu beobachten.

Die erfindungsgemäßen Zubereitungen erfassen somit ein Keimspektrum bei niedrigen minimalen Hemmkonzentrationen, die von den Einzelkomponenten nicht erreicht werden.

Aus den genannten Gründen ist das erfindungsgemäße Kombinationspräparat den Einzelkomponenten bei der Behandlung bakterieller Infektionen überlegen. Es erlaubt, geringere Dosierungen der Einzelkomponenten zuzuführen und dennoch einen besseren Therapieerfolg zu erzielen.

Darüber hinaus ist das erfindungsgemäße Kombinationspräparat anderen antibakteriellen Kombinationspräparaten, wie z.B. der Kombination Cefpirom/Vancomycin (vgl. Beispiel 1) überlegen.

Die Erfindung betrifft auch ein Verfahren zur Herstellung eines pharmazeutischen Kombinationspräparates, das dadurch gekennzeichnet ist, daß man

a) ein Cephalosporinderivat oder dessen physiologisch verträgliche Salze oder Ester und

b) Decaplanin oder dessen physiologisch verträgliche Salze zusammen mit physiologisch annehmbaren Trägern und gegebenenfalls weiteren Hilfs- und Zusatzstoffen in eine geeignete Darreichungsform bringt.

So sind z.B. injizierbare Lösungen, vorzugsweise isotonische wäßrige Lösungen oder Suspensionen, die sterilisiert sein können und Hilfsstoffe, wie Konservierungs-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Kombinationspräparate, die, wenn erwünscht, weitere chemotherapeutisch wertvolle Stoffe enthalten können, werden z.B. mittels konventioneller Verfahren hergestellt.

Die erfindungsgemäßen Kombinationspräparate werden z.B. enteral oder parenteral verabreicht, wobei die parenterale Verabreichung bevorzugt ist.

Das erfindungsgemäße Kombinationspräparat kann zur parenteralen Applikation vorzugsweise unmittelbar vor der Anwendung in sterilem Wasser oder - falls erforderlich -einer Pufferlösung, wie beispielsweise einem Phosphat- oder Carbonatpuffer, wie er üblicherweise für diese Zwecke zum Einsatz kommt, gelöst und anschließend appliziert werden.

Die Dosen des Cephalosporinderivates und von Decaplanin werden in den erfindungsgemäßen Zubereitungen vorzugsweise so gewählt, daß die Einzelkomponenten noch keine ausreichende oder volle Wirkung gegenüber Methicillin-resistenten Staphylokokken-Stämmen zeigen würden. Die Tagesdosis der erfindungsgemäßen Kombination (Summe der Einzelkomponenten) liegt zwischen etwa 1 bis 16 g, vorzugsweise etwa 4 bis 8 g. Das Verhältnis der Einzelkomponenten in der Kombination kann zwischen etwa 1:9 und 9:1, vorzugsweise zwischen etwa 1:5 und 5:1 liegen. Die Dosis in einer Verabreichungseinheit kann beispielsweise zwischen etwa 50 und 2000 mg gewählt werden.

Es kann jedoch erforderlich sein, von den genannten Dosierungen abzuweichen und zwar in Abhängigkeit von der Art und dem Körpergewicht des zu behandelnden Menschen, der Art und Schwere der Erkrankung, der Art der Zubereitung und der Applikation des Arzneimittels sowie dem Zeitraum bzw. Intervall, innerhalb dessen die Verabreichung erfolgt. So kann es in einigen Fällen ausreichend sein, mit weniger als der obengenannten Menge Wirkstoff auszukommen, während in anderen Fällen die obenangeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die folgenden Beispiele dienen zur Erläuterung der Erfindung, ohne daß diese darauf beschränkt wäre.

**Beispiel 1**

Die antibakterielle Aktivität der erfindungsgemäßen Kombinationspräparate wurde für die Kombination Decaplanin + Cefpirom im Agarverdünnungstest mit Müller-Hinton-Agar (Inokulumgröße 5 x $10^4$ keimbildende Einheiten/Impfpunkt) bestimmt.
Kombinationsexperimente wurden nach der Schachbrettmethode durchgeführt. Die "fractional inhibitory concentrations" (FIC-Werte) wurden nach der Methode von Berenbaum bestimmt (M.G. Berenbaum, J. Infect. Dis. 137, (1978), 122 - 130). Definition von Synergismus: Eine Kombination von 2 Substanzen wird gewöhnlich als synergistisch bezeichnet, wenn beide Komponenten zusammen den Teststamm bei einer Konzentration hemmen, die höchstens 1/4 der MHK der Komponente allein ist (C.W. Norden et al., J. Infect. Dis. 140 (1979), 624-633). Bei Anwendung dieses Kriteriums auf die Methode der "fractional inhibitory concentrations" (FIC) entspricht ein FIC-Wert von < 0,5 Synergismus. Höhere FIC-Werte stehen für eine additive Wirkung.

Wie die Tabelle 1 zeigt, weist eine Kombination von Cefpirom mit Decaplanin überraschenderweise für alle Stämme eine synergistische Aktivität auf, während durch die Kombination von Cefpirom mit Vancomycin 9 der 19 Stämme mit einem FIC-Wert >0,5 nicht synergistisch beeinflußt werden. Auch der durchschnittliche FIC-Wert liegt für die Kombination Cefpirom/Decaplanin wesentlich niedriger als für Cefpirom/Vancomycin.

Tabelle 1/B

| | Cefpirom/Decaplanin | Cefpirom/Vancomycin |
|---|---|---|
| <0,5 | 16 Stämme | 4 Stämme |
| =0,5 | 3 Stämme | 6 Stämme |
| >0,5 - 0,75 | 0 Stämme | 8 Stämme |
| >0,75 | 0 Stämme | 1 Stamm |
| ∅ FIC | 0,275 | 0,558 |

Tabelle 2 zeigt die FIC-Werte verschiedener Cephalosporinderivate in Kombination mit Decaplanin für bestimmte Staphylococcus aureus Stämme. Alle Präparate zeigen synergistische Wirkung.

**Tabelle 2**

| | Staphylococcus aureus | | | |
|---|---|---|---|---|
| Präparat | 692 E | 697 E | 706 E | 711a E |
| Cefodizim | 0,141 | 0,313 | 0,25 | 0,188 |
| Cefquinom | 0,25 | 0,375 | 0,141 | 0,375 |
| Ceftazidim | 0,156 | 0,313 | 0,313 | 0,156 |
| Cefuroxim | 0,188 | 0,375 | 0,313 | 0,141 |
| Cefotiam | 0,188 | 0,281 | 0,25 | 0,141 |
| Ceftriaxon | 0,141 | 0,375 | 0,094 | 0,047 |
| Ceftizoxim | 0,25 | 0,25 | 0,047 | 0,062 |
| Cefepim | 0,188 | 0,25 | 0,188 | 0,281 |
| * E 1040 | 0,25 | 0,375 | 0,188 | 0,047 |
| ** DQ 2556 | 0,25 | 0,25 | 0,375 | 0,313 |

Fortsetzung von Tabelle 2

| | Staphylococcus aureus | | | |
|---|---|---|---|---|
| Präparat | 692 E | 697 E | 706 E | 711a E |
| Cefotaxim | 0,188 | 0,313 | 0,25 | 0,125 |
| Cefoperazon | 0,188 | 0,266 | 0,188 | 0,078 |

FIC (Nach Berenbaum)

* (6R,7R(-7-[(5-Amino-1,2,4-thiadiazol-3-yl)-(z)methoxy-imino-acetamido]-3-[4-carbamoyl-1-chinuclidiniomethyl]-8-oxo-5-thia-1-azabicyclo[4.2.0]oct-2-en-2-carboxylat

** 7-[(2-Amino-thiazol-4-yl)-methoxyimino-acetamido]-3-[4-(oxazol-5-yl)-1-pyridinio methyl]-8-oxo-5-thio-1-aza-bicyclo[4.2.0]oct-en-2-carboxylat

**Beispiel 2**

Der überraschend gefundene synergistische Effekt einer Kombination von Decaplanin und Cefpirom konnte auch mit 39 klinischen Isolaten (Methicillin-resistente Staphylokokken) gezeigt werden, von denen die meisten eine ausgeprägte Resistenz gegenüber Cefpirom zeigten (MHK > 16 µg/ml). Dazu wurde die minimale Hemmkonzentration im Agarverdünnungstest von Cefpirom, Decaplanin und einer Mischung gleicher Anteile von Cefpirom und Decaplanin bestimmt. Wie Fig. 1 zeigt, ist die antibakterielle Aktivität der Mischung deutlich höher als die jeder Einzelkomponente. Aus Fig. 1 geht hervor, daß die als Beurteilungskriterium üblicherweise herangezogene minimale Hemmkonzentration, bei der 90 % der Stämme gehemmt werden ($MHK_{90\%}$), für die Mischung beider Komponenten mit 0,7 µg/ml am niedrigsten ist. ($MHK_{90\%}$ für Decaplanin: 3,5 µg/ml, $MHK_{90\%}$ für Cefpirom: 64 µg/ml).

**Beispiel 3**

Herstellung einer parenteralen Zubereitung

1,5 g Cefpirom und 0,5 g Decaplanin werden in 10 ml Wasser ad injec. gelöst und anschließend appliziert.

**Ansprüche**

1. Pharmazeutisches Kombinationspräparat, enthaltend Decaplanin, eine Verbindung der Formel A

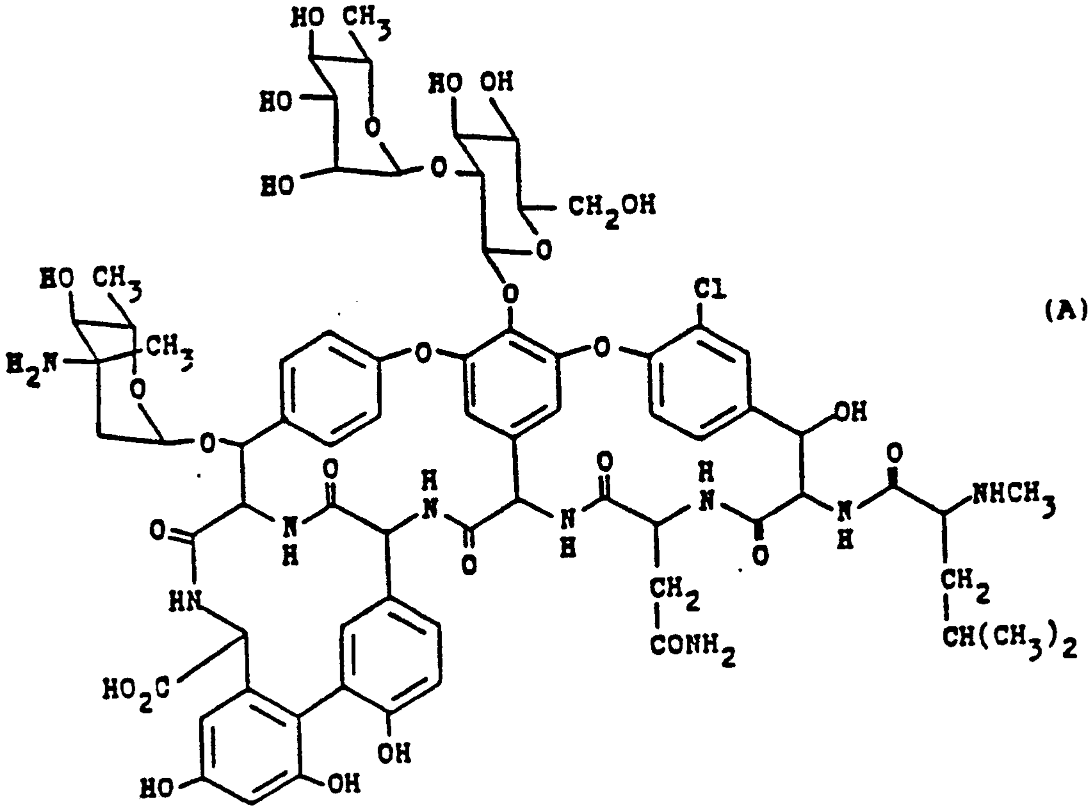

(A)

oder deren physiologisch verträgliche Salze und ein Cephalosporinderivat oder dessen physiologisch verträgliche Salze oder Ester.

2. Pharmezeutisches Kombinationspräparat gemäß Anspruch 1, dadurch gekennzeichnet, daß das Cephalosporinderivat eine Verbindung der Formel I ist,

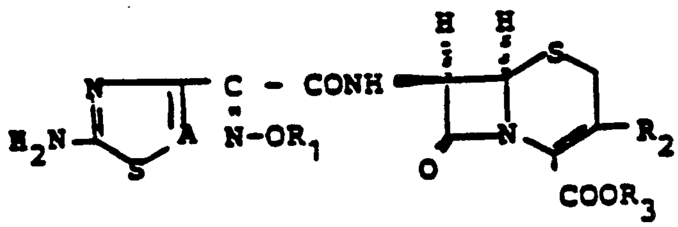

(I)

worin A CH oder N bedeutet und

$R_1$ für Wasserstoff, $C_1$-$C_4$-Alkyl, Carboxyl-$C_1$-$C_4$-alkyl oder eine Gruppe der Formel

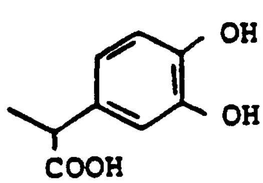

und die Gruppe $=N\text{-}OR_1$ in syn-Position steht,

$R_2$ die Bedeutung haben kann von Wasserstoff, Methyl, Methoxy, Vinyl, Acetoxymethyl, Carbamoyloxymethyl, von

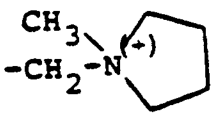 , 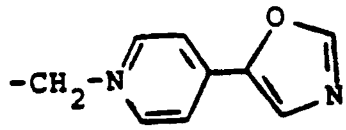 ,

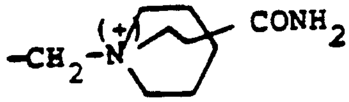

oder von

$-CH_2S-X,$

mit X = 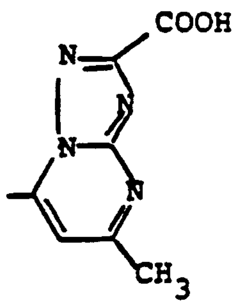 , 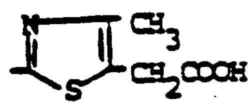 , 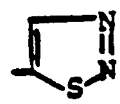 ,

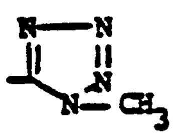 , 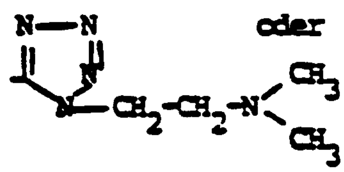 oder 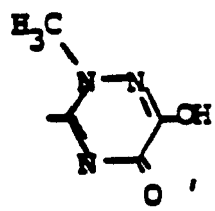 ,

von 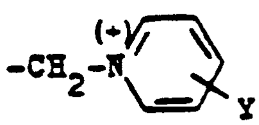 mit Y = Wasserstoff, $C_1$-$C_4$-Alkylthio, $C_1$-$C_4$-Alkoxy oder $C_3$-$C_5$-Cycloalkyl,

von 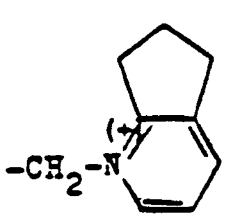 oder 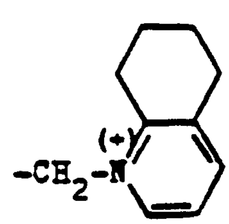

wobei die ankondensierten Ringe auch in 3,4-Stellung stehen und auch durch Sauerstoff unterbrochen sein können, von

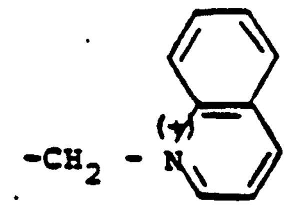

oder

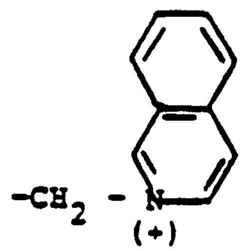

von Thienopyridinio-methyl, Furopyridinio-methyl oder von 5-Methyl-tetrazol-2-yl-methyl und $R_3$ für Wasserstoff, ein physiologisch verträgliches Kation, einen physiologisch verträglichen Esterrest oder - falls in $R_2$ die Struktur

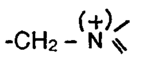 auftritt - für eine negative Ladung stehen kann.

3. Pharmazeutisches Kombinationspräparat gemäß Anspruch 1 oder 2, dadurch gekennzeichnet, daß das Cephalosporinderivat ausgewählt ist aus der Gruppe der folgenden Verbindungen: Cefotaxim, Cefixim, Cefodizim, Ceftriaxon, Cefmenoxim, Cefpirom, Cefotiam, Cefoperazon, Cefepim, Cefquinom, Cefuroxim, Ceftizoxim, Ceftazidim, 7-[(2-Aminothiazol-4-yl)-methoxyimino-acetamido]-3-[4-(oxazol-5-yl)-1-pyridiniomethyl)-8-oxo-5-thia-1-azabicyclo[4.2.0]oct-2-en-2-carboxylat, (6R,7R)-7-[(2-Amino-thiazol-4-yl)-((Z)-(S)-α-carboxy-3,4-dihydroxy-benzyloxyimino)-acetamido]-3-[(2-carboxy-5-methyl-S-triazolo[1,5-a]pyrimidin-7-yl)-thiomethyl]-8-oxo-5-thia-1-azabicyclo[4.2.0]oct-2-en-2-carbonsäure und (6R,7R)-7-[(5-Amino-1,2,4-thiadiazol-3-yl)-(Z)-methoxyimino-acetamido]-3-[4-carbamoyl-1-chinuclidiniomethyl]-8-oxo-5-thia-1-azabicyclo[4.2.0]-oct-2-en-2-carboxylat.

4. Pharmazeutisches Kombinationspräparat gemäß einem der Ansprüche 1 - 3, dadurch gekennzeichnet, daß das Cephalosporinderivat Cefpirom, eine Verbindung der Formel

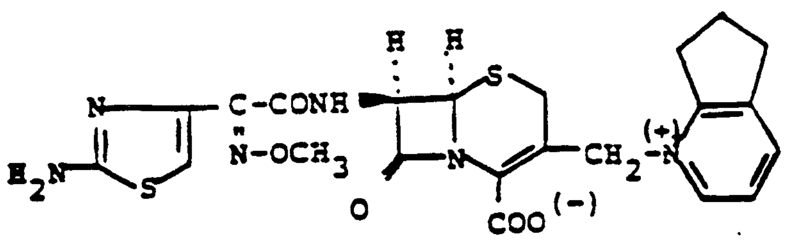

ist.

5. Verfahren zur Herstellung eines pharmazeutischen Kombinationspräparates gemäß einem oder mehreren der Ansprüche 1 - 4, dadurch gekennzeichnet, daß man Decaplanin oder dessen physiologisch verträgliche Salze und ein Cephalosporinderivat oder dessen physiologisch verträgliche Salze oder Ester zusammen mit physiologisch verträglichen Träger- und gegebenenfalls weiteren Hilfs-und/oder Zusatzstoffen in eine geeignete Darreichungsform bringt.

6. Verwendung eines pharmazeutischen Kombinationspräparates gemäß einem oder mehreren der Ansprüche 1 - 4 zur Prophylaxe oder Behandlung von bakteriellen Infektionskrankheiten.

7. Ein pharmazeutisches Kombinationspräparat gemäß einem oder mehreren der Ansprüche 1 - 4 als Mittel zur Prophylaxe oder Behandlung von bakteriellen Infektionskrankheiten.

Patentansprüche für folgenden Vertragsstaaten: ES, GR

1. Verfahren zur Herstellung eines pharmazeutischen Kombinationspräparates, dadurch gekennzeichnet, daß man Decaplanin, eine Verbindung der Formel A

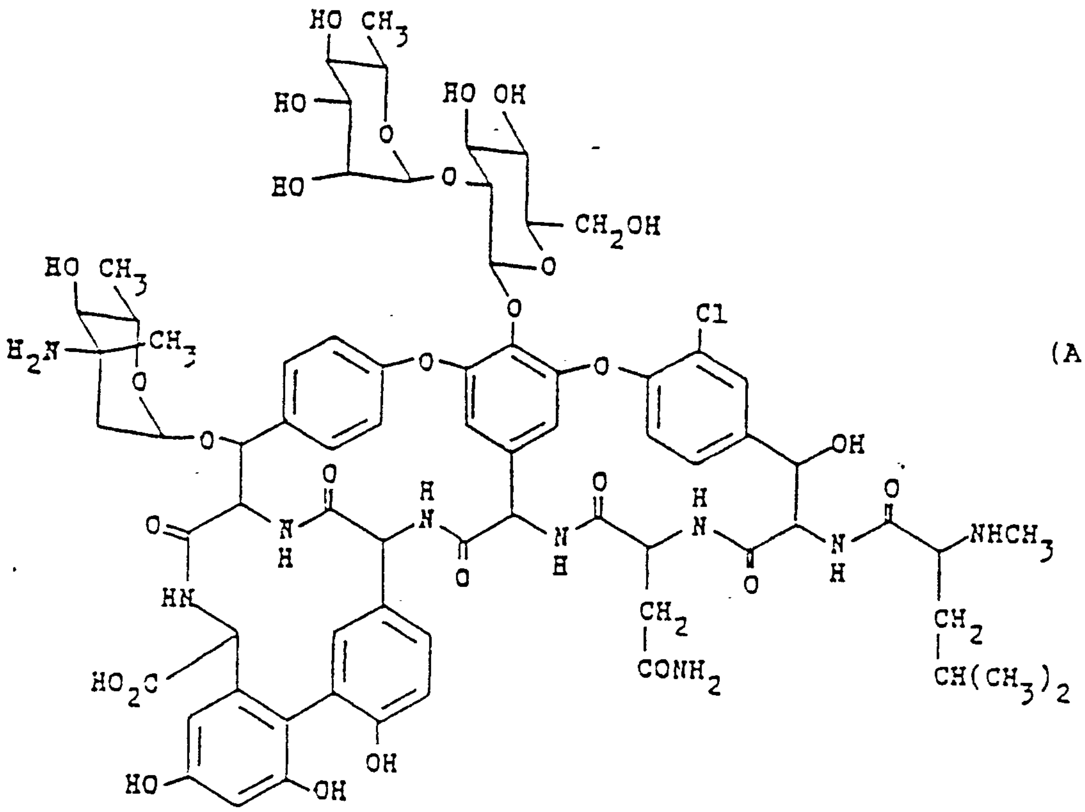

(A)

oder deren physiologisch verträgliche Salze mit einem Cephalosporinderivat oder dessen physiologisch verträglichen Salzen oder Estern, gegebenenfalls mit physiologisch verträglichen Träger-, Hilfs- und/oder Zusatzstoffen in eine geeignete Darreichungsform bringt.

2. Verfahren gemäß Anspruch 1, dadurch gekennzeichnet, daß das Cephalosporinderivat eine Verbindung der Formel I ist,

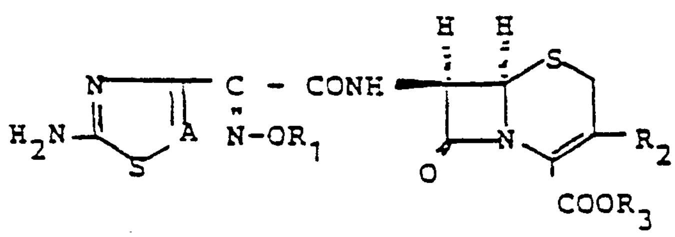

(I)

worin A CH oder N bedeutet und

$R_1$ für Wasserstoff, $C_1$-$C_4$-Alkyl, Carboxyl-$C_1$-$C_4$-alkyl oder eine Gruppe der Formel

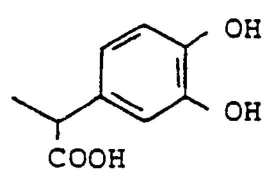

und die Gruppe $=N\text{-}OR_1$ in syn-Position steht,

$R_2$ die Bedeutung haben kann von Wasserstoff, Methyl, Methoxy, Vinyl, Acetoxymethyl, Carbamoyloxymethyl, von

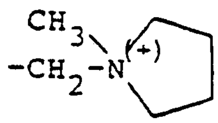, 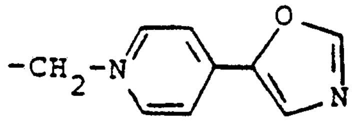,

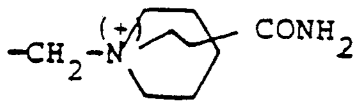

oder von

$-CH_2S-X$,

mit X = 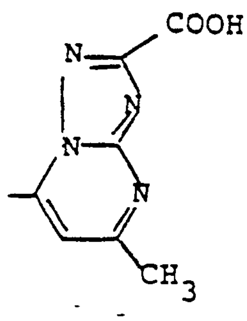, 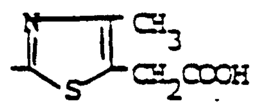, 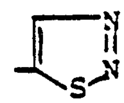,

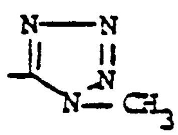, 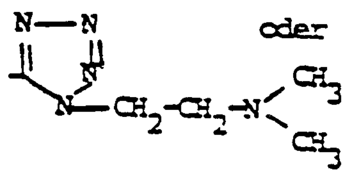 oder 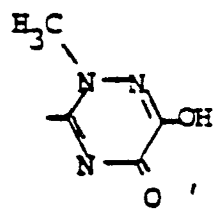,

von 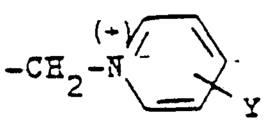 mit Y = Wasserstoff,
$C_1$-$C_4$-Alkylthio,
$C_1$-$C_4$-Alkoxy oder
$C_3$-$C_5$-Cycloalkyl,

von 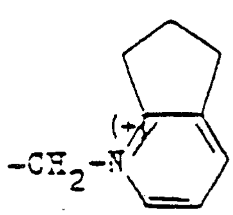 oder 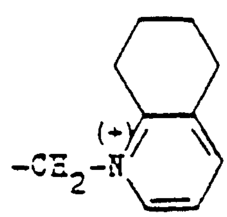

wobei die ankondensierten Ringe auch in 3,4-Stellung stehen und auch durch Sauerstoff unterbrochen sein

können, von

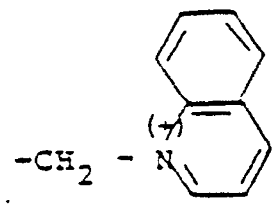

oder

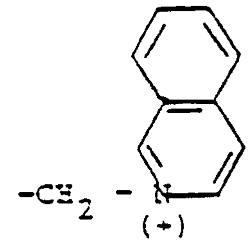

von Thienopyridinio-methyl, Furopyridinio-methyl oder von 5-Methyl-tetrazol-2-yl-methyl und $R_3$ für Wasserstoff, ein physiologisch verträgliches Kation, einen physiologisch verträglichen Esterrest oder - falls in $R_2$ die Struktur $-CH_2-N$ auftritt - für eine negative Ladung stehen kann.

3. Verfahren gemäß Anspruch 1 oder 2, dadurch gekennzeichnet, daß das Cephalosporinderivat ausgewählt ist aus der Gruppe der folgenden Verbindungen: Cefotaxim, Cefixim, Cefodizim, Ceftriaxon, Cefmenoxim, Cefpirom, Cefotiam, Cefoperazon, Cefepim, Cefquinom, Cefuroxim, Ceftizoxim, Ceftazidim, 7-[(2-Aminothiazol-4-yl)-methoxyimino-acetamido]-3-[4-(oxazol-5-yl)-1-pyridiniomethyl]-8-oxo-5-thia-1-azabicyclo[4.2.0]oct-2-en-2-carboxylat, (6R,7R)-7-[(2-Amino-thiazol-4-yl)-((Z)-(S)-α-carboxy-3,4-dihydroxy-benzyloxyimino)-acetamido]-3-[(2-carboxy-5-methyl-5-triazolo[1,5-a]pyrimidin-7-yl)thiomethyl]-8-oxo-5-thia-1-azabicyclo[4.2.0]oct-2-en-2-carbonsäure und (6R,7R)-7[(5-Amino-1,2,4-thiadiazol-3-yl)-(Z)-methoxyimino-acetamido]-3-[4-carbamoyl-1-chinuclidiniomethyl]-8-oxo-5-thia-1-azabicyclo[4.2.0]oct-2-en-2-carboxylat.

4. Verfahren gemäß einem der Ansprüche 1 - 3, dadurch gekennzeichnet, daß das Cephalosporinderivat Cefpirom, eine Verbindung der Formel

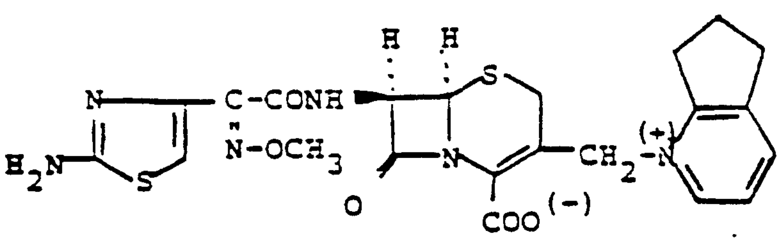

ist.

5. Verwendung eines pharmazeutischen Kombinationspräparates gemäß einem oder mehreren der Ansprüche 1 - 4 zur Prophylaxe oder Behandlung von bakteriellen Infektionskrankheiten.

6. Verfahren zur Prophylaxe oder Behandlung von bakteriellen Infektionskrankheiten, dadurch gekennzeichnet, dar ein Kombinationspräparat hergestellt gemäß einem oder mehreren der Ansprüche 1 - 4 verabreicht wird.

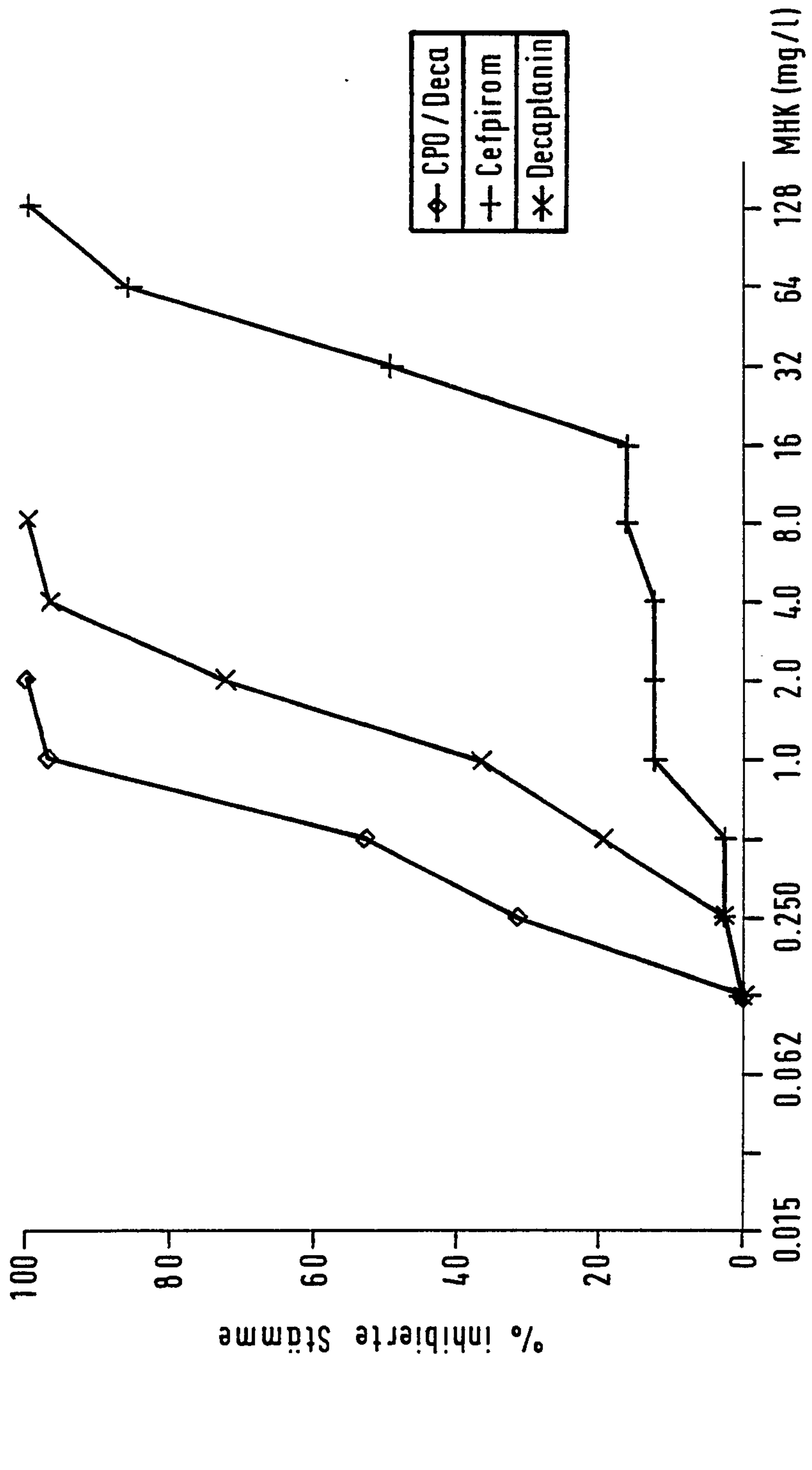


Antibakterielle Aktivität in vitro von Cefpirom, Decaplanin und einer Mischung gleicher Anteile beider Substanzen gegen 39 klinische Isolate Methicillin-resistenter Staphylokokken

Europäisches Patentamt

# EUROPÄISCHER RECHERCHENBERICHT

Nummer der Anmeldung

EP 89 11 5520

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | THE JOURNAL OF ANTIBIOTICS, Band XLII, Nr. 4, April 1989, Seiten 497-505; E. RIVA et al.: "A42867, a novel glycopeptide antibiotic"<br>----- | | A 61 K 37/02 |
| | | | RECHERCHIERTE SACHGEBIETE (Int. Cl.5) |
| | | | A 61 K |

Der vorliegende Recherchenbericht wurde für alle Patentansprüche erstellt

| Recherchenort | Abschlußdatum der Recherche | Prüfer |
|---|---|---|
| DEN HAAG | 05-07-1990 | NOVOA Y SANJURJO M.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)